# FASCICULE DE BREVET EUROPEEN

(11) **EP 0 647 617 B1**
(45) Date de publication et mention de la délivrance du brevet: **23.07.1997**
(21) Numéro de dépôt: 94402272.2
(22) Date de dépôt: 11.10.1994
(51) Int. Cl.: C07C 235/08, C07C 235/34, A61K 7/48

(54) **Céramides, leur procédé de préparation et leurs applications en cosmétique**
Ceramide, Verfahren zu deren Herstellung und deren Verwendungen in der Kosmetik
Ceramides, process for their preparation and their cosmetic uses

(30) Priorité: 12.10.1993 FR 9312106
(43) Date de publication de la demande: 12.04.1995
(73) Titulaire: L'OREAL, 75008 Paris (FR)
(72) Inventeur: Semeria, Didier, F-77181 Courtry (FR); Philippe, Michel, F-92160 Chatou (FR); Mahieu, Claude, F-75017 Paris (FR)
(74) Mandataire: Casalonga, Axel

(56) Documents cités:
- EP-A- 0 373 038
- EP-A- 0 482 860
- EP-A- 0 500 437
- CHEMICAL ABSTRACTS, vol. 107, no. 20, 16 Novembre 1987, Columbus, Ohio, US; abstract no. 183350, HATTORI, MICHIHIRO ET AL 'Cosmetics containing lipids and surfactants for skin conditioning'
- DATABASE WPI Derwent Publications Ltd., London, GB; AN 86-344267 & JP-A-61 260 008 (SUNSTAR KK)
- JOURNAL OF AGRICULTURAL AND FOOD CHEMISTRY, vol.39, 1991, WASHINGTON US pages 1709 - 1714 MICHIYUKI KOJIMA ET AL. 'Composition and molecular Species of Ceramide and Cerebroside in Scarlet Runner Beans (Phaseolus Coccineus L.) and Kidney Beans (Phaseolus Vulgaris L.)'
- CHEMICAL ABSTRACTS, vol. 115, no. 7, 19 Août 1991, Columbus, Ohio, US; abstract no. 68389, KOJIMA, MICHIYUKI ET AL. 'Chemical Characterization of lipids in suspension cell cultures from several organs of Adzuki beans.'
- J. LIPID RES., vol.30, no.4, 1989 pages 616 - 627 HIROKO KADOWAKI ET AL. 'Separation of derivatized glycosphingolipids into individual molecular species by high performance liquid chromatography'

## Description

La présente invention a pour objet de nouveaux céramides, leur procédé de préparation ainsi que leur utilisation, notamment pour les traitements et les soins de la peau et des cheveux en cosmétique ou en dermopharmacie.

L'exposition de la peau au froid, au soleil, aux atmosphères à faible humidité relative, les traitements répétés avec des compositions de lavage ou encore le contact avec des solvants organiques, sont des facteurs qui entraînent, à des degrés divers, un déssèchement apparent. La peau apparaît plus sèche, moins souple et le relief cutané plus prononcé. Par ailleurs, les cheveux, qui sont soumis trop fréquemment à certains traitements capillaires, perdent leur aspect brillant et peuvent devenir rèches et cassants.

La demanderesse a donc recherché des composés qui permettent de prévenir ou de corriger ces phénomènes se traduisant par un déssèchement apparent et qui redonnent à la peau sa souplesse et aux cheveux leur brillance et leur douceur.

Pour résoudre ce problème, on a déjà proposé d'utiliser des céramides. On sait en effet que ces composés sont les éléments constitutifs prépondérants des lipides intercornéocytaires du stratum cornéum et participent au maintien de l'intégrité de la barrière cutanée. Ils représentent, selon DOWNING ("The Journal of Investigative Dermatology", vol. 88, n° 3, p. 25-65, supplément mars 1987), environ 40% de la totalité de ces lipides.

Les céramides utilisés en cosmétique sont des extraits naturels issus notamment de la peau de porc, du cerveau de boeuf, de l'oeuf, des cellules du sang, des plantes etc... (brevets JP 61260008 et JP 62120308). De tels céramides ont également été proposés pour la protection des cheveux (EP 0278 505).

Il s'agit donc toujours de mélanges de teneur plus ou moins importante en céramides et dont la composition est difficile à contrôler. De plus, ces mélanges sont sujets à la contamination bactérienne. Leur conservation est très difficile à maîtriser. Lorsqu'ils sont d'origine animale, il y a en plus un risque de contamination par l'agent responsable de la BSE (encéphalopathie bovine spongiforme).

Pour résoudre ces problèmes, on a proposé des céramides de synthèse, notamment dans la demande de brevet français n° 2 673 179. Plus particulièrement, cette demande décrit des céramides de synthèse de formule : 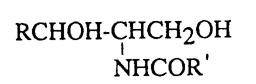 dans laquelle R désigne un radical alcoyle ou alcènyle en C₁₁ à C₂₁, R' désigne un radical hydrocarboné en C₁₁-C₁₉ linéaire et portant une ou plusieurs insaturations éthyléniques ou un mélange de radicaux hydrocarbonés en C₁₁-C₁₉, linéaires, saturés ou portant une ou plusieurs insaturations éthyléniques, dans lequel la proportion de radicaux saturés n'excède pas 35 %, ces composés étant sous forme de mélange racémique des diastéréoisomères érythro et thréo dans les proportions érythro : thréo de 85 : 15 à 60 : 40.

Ces composés utilisés dans des compositions cosmétiques ou dermopharmaceutiques, pour les traitements et les soins de la peau et des cheveux ont un effet hydratant permettant de prévenir ou de corriger certains effets du dessèchement apparent de la peau ou des cheveux.

Toutefois, il serait encore souhaitable de mettre au point des composés qui, utilisés dans des compositions cosmétiques ou dermatologiques, aient un effet hydratant supérieur à celui des composés de la demande de brevet français n° 2 673 179.

La demande de brevet WO 93/02 656 décrit des dispersions cationiques contenant au moins un céramide ou glycocéramide ou un mélange de céramides et/ou glycocéramides naturels ou synthétiques dont la chaîne N-acylante est une chaîne saturée ou insaturée pouvant comporter un groupement hydroxyle en position alpha par rapport au carbonyle. Ces dispersions aqueuses à base de céramides et/ou glycocéramides lorsqu'ils sont associés à des agents tensio-actifs cationiques particuliers, améliorent le démêlage des cheveux sans les alourdir ni les graisser.

La présente invention a donc pour objet de nouveaux composés ayant un meilleur pouvoir hydratant de la peau et/ou des cheveux lorsqu'ils sont utilisés dans des compositions cosmétiques ou dermopharmaceutiques.

Les nouveaux composés selon l'invention, répondent à la formule : 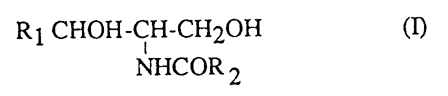 dans laquelle :
- R₁ désigne un radical alkyle ou alcényle en C₁₁ à C₂₁;
- R₂ désigne un radical hydroxyalkyle linéaire ou ramifié en C₁ à C₂₉, ou un groupement hydroxyaralkyle en C₇ à C₂₉, le groupement hydroxy étant en position alpha du carbonyle, ces composés étant sous forme d'un mélange racémique des diastéréoisomères érythro et thréo, pour la partie aminodiol 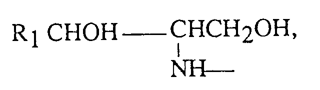
dans les proportions érythro : thréo de 85 : 15 à 20 : 80, de préférence 65:35 à 45:55.

De préférence R₁ désigne un radical alkyle ou alcényle en C₁₃ à C₁₉ et en particulier le radical pentadécyle.

De préférence R₂ est un radical 1-hydroxyalkyle linéaire en C₁ à C₂₁, plus particulièrement en C₁₅ à C₂₁, notamment les radicaux 1-hydroxy pentadécyle et 1-hydroxyhénéicosyle ou un groupement 1-hydroxyaralkyle en C₇ à C₁₉, notamment le groupement D, L-1-hydroxy benzyle.

Les composés de formule (I) selon l'invention sont des cires particulièrement utiles lorsqu'un effet de lutte contre le déssèchement de la peau ou des cheveux est recherché en cosmétique ou en dermopharmacie.

En particulier les composés selon l'invention présentent une forte activité dans l'essai de perte insensible en eau (PEI) qui est une mesure de l'effet de barrière et une bonne activité dans l'essai "Dermodiag" qui est un essai de mesure de l'hydratation, alors que des composés analogues, mais qui ne se présentent pas sous la forme d'un mélange racémique des diastéréoisomères érythro et thréo présentent une très faible activité dans l'essai PEI et pas d'activité dans l'essai "Dermodiag", et les composés, même sous forme d'un mélange racémique des diastéréoisomères érythro et thréo, mais ne comportant pas de chaîne N-acylante α-hydroxylée, ne présentent pas d'activité dans l'essai "Dermodiag".

L'essai "Dermodiag" est un mesure de la conductivité de la peau, liée à son taux d'hydratation. Dans cet essai, on utilise un appareil "Dermodiag" destiné à mesurer le taux d'hydratation des couches supérieures de l'épiderme. Du point de vue électrique, la peau se comporte comme une résistance en parallèle avec une capacité. Ces deux éléments (surtout la capacité équivalente) sont très dépendants de la teneur en eau des cellules.

L'appareil utilise la peau comme condensateur de bouclage et se met à osciller à des fréquences variables selon la quantité d'eau dans les tissus supérieurs. Ces phénomènes se passent à haute fréquence (de l'ordre de plusieurs MHz).

L'appareil comprend deux électrodes concentriques posées sur la peau, et les lignes de champs électriques se referment entre ces deux électrodes. L'affichage digital sur l'appareil indique alors le courant consommé à chaque mesure. Plus le taux d'hydratation est important, plus la valeur affichée est grande.

Ces composés présentent par ailleurs une faible agressivité vis-à-vis de la peau ou des muqueuses oculaires et une bonne tolérance vis-à-vis des membranes cellulaires comme celles des érythrocytes.

Les nouveaux composés de formule (I) ci-dessus présentent des propriétés émollientes et adoucissantes. Ils sont facilement solubilisés dans les phases grasses des préparations cosmétiques ou dermopharmaceutiques.

Les cheveux traités par ces composés présentent un aspect brillant et une moins grande sensibilité à l'eau, due à l'apport de matière lipidique uniformément répartie sur les écailles du cheveu. Les propriétés mécaniques et de nervosité sont également améliorées.

Ces composés forment, en association avec d'autres lipides, des vésicules.

Les céramides de formule (I) ci-dessus sont obtenus par acylation de la fonction amine d'une sphingosine ou d'une sphinganine ou d'un dérivé réactif de celles-ci, tel que par exemple le chlorhydrate, avec un agent acylant approprié.

Dans la présente invention, on entendra par sphingosine ou sphinganine les composés D, L, c'est-à-dire, les mélanges racémiques des diastéréoisomères érythro et thréo.

Un autre objet de la présente invention concerne donc un procédé de préparation des composés de formule (I) qui peut être représenté par le schéma suivant :

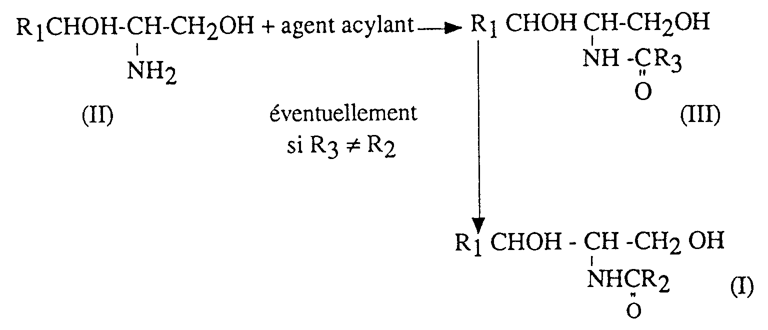

Plus particulièrement, les composés de formule (I) ci-dessus peuvent être obtenus par acylation, en milieu anhydre ou dans un solvant approprié, de la fonction amine d'une sphingosine ou d'une sphinganine de formule (II) ci-dessus, dans laquelle R₁ est tel que défini précédemment, avec un agent acylant choisi parmi les chlorures d'acides, les anhydrides d'acide, les anhydrides mixtes, les esters de paranitrophénol, les esters de succinimide, les esters de carbodiimide, les esters d'alkyle inférieur en C₁ à C₈, les azolides, notamment les imidazolides et les pyrazolides, et les O-carboxyanhydrides des 2-hydroxy acides correspondants appropriés.

Plus particulièrement, l'agent acylant est choisi parmi les O-carboxyanhydrides des acides 2-hydroxy correspondants et les composés de formule R₃COA (IV) dans laquelle :
R₃ est choisi parmi le radical R₂ défini ci-dessus, les radicaux alkyles linéaires ou ramifiés en C₁-C₂₉, de préférence linéaires en C₁ à C₂₁, et aralkyles en C₇ à C₂₉, de préférence en C₇ à C₁₉, substitués en position α par rapport au carbonyle par un substituant choisi parmi -Br, -Cl, -I et -OB, où OB est un groupe susceptible de former un groupe -OH ; et
A est choisi parmi les halogènes, 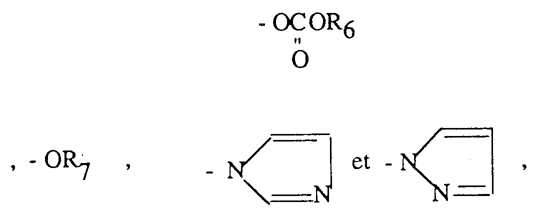 où
R₆ est un radical alkyle inférieur en C₂ à C₈ et R₇ est choisi parmi les radicaux alkyles inférieurs en C₁ à C₈, 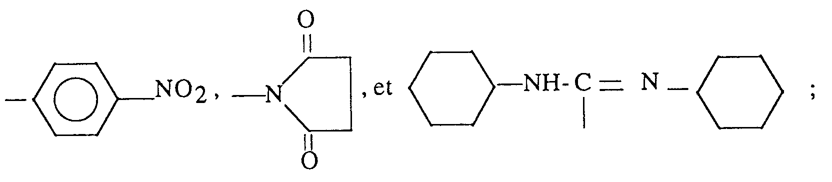 à la condition que lorsque R₃ représente le radical R₂, A soit différent de -Cl.

Des agents acylants recommandés sont les esters de succinimide et de carbodiimide.

Selon la nature de l'agent acylant utilisé, la réaction d'acylation du groupe amine du composé de formule (II) s'effectuera à l'état anhydre ou en présence d'un solvant.

Parmi les solvants utiles dans le procédé de la présente invention on peut citer, le tétrahydrofurane, la pyridine, le diméthylformamide, le dichlorométhane et le tertiobutylméthyl éther.

De préférence le groupement -OB est choisi parmi les radicaux suivants : acétate, benzoate, benzyloxy,
-OSi(CH₃)₃, -OSi(CH₃)₂(t-butyl), et
-OSi(t-butyl)(-C₆H₅)₂.

De préférence, -OB est un groupement acétate.

De préférence, A est choisi parmi les radicaux suivants : 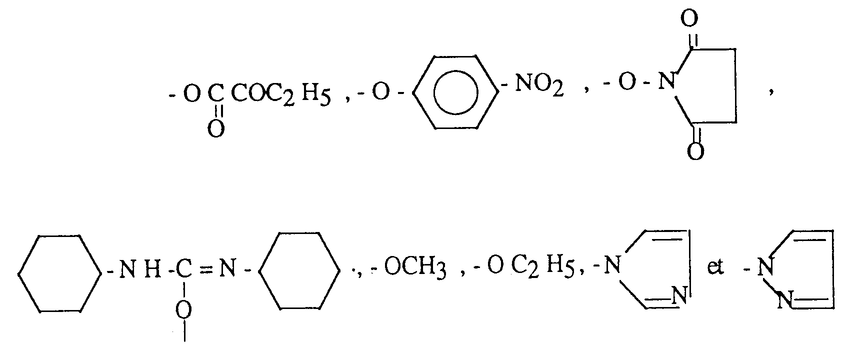 On recommande tout particulièrement pour A les radicaux

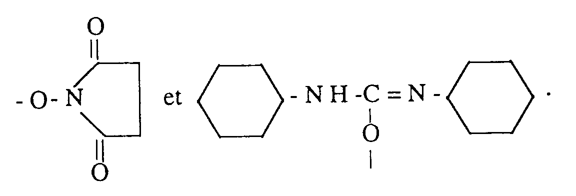

Des agents acylants particulièrement recommandés sont le 2-hydroxyhexadécanoate de succinimide, le 2-hydroxyhexadécanoate de dicyclohexylcarbodiimide, le 2-hydroxydocosanoate de succinimide, le 2-hydroxydocosanoate de dicyclohéxylcarbodiimide et le D, L mandélate de succinimide.

Bien évidemement, lorsque R₃ est différent de R₂ et représente un radical alkyle en C₁ à C₂₉ ou aralkyle en C₇ à C₂₉, substitué en position α par rapport au carbonyle comme indiqué précédemment, une ou plusieurs étape(s) supplémentaire(s) est (sont) nécessaire(s) pour transformer le substituant en position α par rapport au carbonyle en un radical hydroxyle pour obtenir le composé de formule (I). Cette transformation est bien connue et peut se faire par exemple par hydrolyse.

On peut également, selon l'invention préparer les composés de formule (III) pour lesquels R₃ = R₂ en faisant réagir un composé de formule (II) avec un O- carboxyanhydride de l'acide 2-hydroxy correspondant.

Les O-carboxyanhydrides sont des composés connus, qui peuvent être obtenus par des techniques connues en faisant réagir l'acide 2-hydroxyle correspondant avec du phosgène ou un de ses substituants di ou triphosgène. Les O-carboxyanhydrides et leurs procédés de préparation sont décrit, en particulier, par K. TOYOOKA, dans Heterocycles vol. 29, n° 5, pages 975-978 (1989).

Les réactions d'acylation avec un ester d'alkyle inférieure se font à l'état anhydre. Elles sont notamment décrites par E.F. JORDAN dans JAOCS p. 600-605 (1961).

Les autres réactions sont effectuées dans des solvants tels que, par exemple, le tétrahydrofurane, la pyridine, le diméthylformamide, et le dichlorométhane.

L'acylation par un ester de succinimide et de dicyclohexylcarbodiimide est décrite notamment par LAPIDOT dans J. Lipid Res. 8, 142-145 (1967).

L'acylation par un ester de paranitrophénol est décrite notamment par BODANSKY dans Nature n° 4459 p. 685 (1955).

L'acylation par un anhydride mixte est décrite par J.L. TORRES dans Tetrahedron vol. 43 n° 17, p. 4031-3 (1987).

Les acylations avec les azolides sont décrites par H.A. STAAB dans Angew, Chem. Internat. Edit. Vol. 1 n° 7 p.357-367 (1962).

Les réactions d'acylation sont décrites en général par J. MARCH dans Advanced Organic Chemistry-Third Edition - JOHN WILEY & SONS-INC p. 370-377 (1985).

Pour la préparation du composé (I) de l'invention, on peut également utiliser le chlorhydrate du composé (II).

Les composés (II) sont des composés connus. Leur synthèse a été décrite en particulier par D. SHAPIRO dans "Chemistry of sphingolipids", HERMANN, Paris (1969).

Quand R₁ désigne un radical alcényle, les composés (II) sous leur formes D,L-érythro sont des sphingosines dont la synthèse est décrite à la page 21 de "Chemistry of Sphingolipids".

Quand R₁ désigne un radical alkyle, les composés (II) sous leur formes D,L-érythro sont des sphinganines ou encore appelées des dihydrosphingosines. Elles peuvent être préparées en particulier à partir de 2- acétamido-3-oxo-alcanoate de méthyle ou d'éthyle, comme décrit dans "Chemistry of Sphingolipids", page 32.

Les procédés de synthèse des sphingosines ou des sphinganines décrits ci-dessus conduisent à des mélanges racémiques des diastéréoisomères érythro et thréo dans les proportions érythro-thréo de 85:15 à 20:80.

Les composés de formule (III), et plus particulièrement lorsque R₃=R₂, issus de la réaction d'acylation, peuvent subir une réaction de protection des groupements hydroxyle, par réaction avec un agent protecteur choisi parmi les anhydrides d'acide, les halogénures d'acide et les chlorosilanes, la réaction étant suivie, après isolation du produit, par une hydrolyse, de préférence en milieu basique.

Les agents protecteurs, utiles dans le procédé de la présente invention, sont de préférence choisis parmi l'anhydride acétique, le chlorure d'acétyle, le chlorure de benzoyle, le chlorure de benzyle, le bromure de benzyle, les chlorosilanes de formule ClSi(CH₃)₃, ClSi(CH₃)₂(tBu), ClSi(tBu)(-C₆H₅)₂.

Les composés selon l'invention peuvent recevoir des applications diverses, notamment en tant que constituants cireux dans des compositions cosmétiques et dermopharmaceutiques. Ces composés possèdent en plus la propriété de former des vésicules en association avec d'autres lipides, lorsqu'ils sont dispersés dans l'eau.

La présente invention a donc pour objet l'utilisation des composés lipidiques de formule (I) en tant que constituants cireux dans des émulsions, des dispersions ou dans des lotions. Elle a également pour objet l'utilisation de ces composés, associés à d'autres lipides, pour la formation de sphérules lipidiques.

La présente invention a également pour objet des compositions à usage cosmétique ou dermopharmaceutique contenant un composé de formule (I).

Un autre objet de l'invention est constitué par un procédé de traitement cosmétique de la peau, des cheveux ou des poils consistant à appliquer sur ces derniers une quantité suffisante d'une telle composition contenant un composé de formule (I).

Les compositions selon l'invention peuvent se présenter sous forme d'émulsions (lait ou crème), de lotions hydroalcooliques, huileuses ou oléoalcooliques, de gels, de dispersions ou de bâtonnets solides, de sprays ou de mousses aérosol.

Selon l'invention, les composés de formule (I) représentent 0,05% à 20%, et de préférence 0, 1 à 10% du poids total de la composition.

Les compositions sont par exemple des lotions, des laits ou des crèmes émollients, des laits ou des crèmes pour les soins de la peau ou des cheveux, des crèmes, des lotions ou des laits démaquillants, des bases de fond de teint, des lotions, des laits ou des crèmes antisolaires, des lotions, des laits ou des crèmes de bronzage artificiel, des crèmes ou des mousses de rasage, des lotions après rasage, des shampooings ou des mascaras.

Ces compositions peuvent également se présenter sous la forme de bâtons pour les lèvres destinés soit à les colorer, soit à éviter les gerçures, ou de produits de maquillage pour les yeux ou de fards et fonds de teint pour le visage.

Lorsque les compositions selon l'invention se présentent sous forme d'émulsions du type eau-dans-l'huile ou huile-dans-l'eau, la phase grasse est essentiellement constituée d'un mélange de composé de formule (I) avec au moins une huile, et éventuellement un autre corps gras.

La phase grasse des émulsions peut constituer de 5 à 60% du poids total de l'émulsion.

La phase aqueuse desdites émulsions constitue de préférence de 30 à 85% du poids total de l'émulsion.

La proportion de l'agent émulsionnant peut être comprise entre 1 et 20%, et de préférence entre 2 et 12% du poids total de l'émulsion.

Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau; les lotions huileuses peuvent en outre constituer des huiles moussantes contenant un tensio-actif oléosoluble, des huiles pour le bain, etc.

Parmi les principaux adjuvants pouvant être présents dans les compositions selon l'invention, on peut citer les corps gras tels que les huiles ou les cires minérales, animales ou végétales, les acides gras, les esters d'acides gras tels que les triglycérides d'acides gras ayant de 6 à 18 atomes de carbone, les alcools gras; les émulsionnants comme les alcools gras oxyéthylénés ou les alcoyléthers de polyglycérol; les solvants tels que les monoalcools ou polyalcools inférieurs contenant de 1 à 6 atomes de carbone ou encore l'eau.

Les mono- ou polyalcools plus particulièrement préférés sont choisis parmi l'éthanol, l'isopropanol, le propylèneglycol, le glycérol et le sorbitol.

A titre de corps gras, parmi les huiles minérales, on peut citer l'huile de vaseline; parmi les huiles animales, les huiles de baleine, de phoque, de menhaden, de foie de flétan, de morue, de thon, de tortue, de pied de boeuf, de pied de cheval, de pied de mouton, de vison, de loutre, de marmotte, etc.; parmi les huiles végétales, les huiles d'amande, de germe de blé, d'olive, de germe de maïs, de jojoba, de sésame, de tournesol, de palme, de noix, de karité, de shoréa, de macadamia, de pépins de cassis et similaires.

Parmi les esters d'acides gras, on peut utiliser des esters d'acides en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire microcristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de Ca, Mg et Al.

Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de polyglycérol, les alcools en C₁₂-C₁₈ comportant de 2 à 10 moles de glycérol.

Il peut aussi être utile d'utiliser des épaississants tels que les dérivés de cellulose, les dérivés d'acide polyacrylique, les gommes de guar ou de caroube ou la gomme de xanthane.

La composition selon l'invention peut également contenir des adjuvants habituellement utilisés en cosmétique ou en dermopharmacie et notamment des produits hydratants, des adoucissants, des produits pour le traitement d'affections cutanées, des filtres solaires, des germicides, des colorants, des conservateurs, des parfums et des propulseurs.

Lorsque les compositions selon l'invention sont des dispersions, il peut s'agir de dispersions de composés de formule (I) dans l'eau en présence de tensio-actif ou encore de dispersions aqueuses de sphérules lipidiques, constituées de couches moléculaires organisées enfermant une phase aqueuse encapsulée, ces couches étant constituées d'au moins un composé de formule (I), associé à au moins un autre composé lipidique.

On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français n° 2 315 991, 1 477 048 et 2 091 516 ou dans les demandes de brevet international WO 83/01 571 et WO92/08685.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. On peut aussi utiliser avantageusement comme lipides formant la phase lamellaire, des éthers de polyglycérol tels que ceux décrits dans les brevets français n° 1 477 048, 2 091 516, 2 465 780 et 2 482 128.

A titre de groupement ionique, on peut avantageusement utiliser un groupement dérivé d'un composé amphotère, anionique ou cationique.

D'autres lipides décrits dans la demande de brevet international WO 83/01 571 comme pouvant être utilisés pour la formation de vésicules sont les glycolipides comme le lactosylcéramide, le galactocérébroside, les gangliosides et le trihexosylcéramide, ainsi que les phospholipides tels que le phosphatidylglycérol et le phosphatidylinositol.

La présente invention a donc également pour objet une dispersion de sphérules lipidiques constituées de couches moléculaires organisées de composé(s) de formule (I) et de lipide défini ci-dessus renfermant une phase aqueuse à encapsuler.

La phase continue de la dispersion qui entoure les sphérules est une phase aqueuse.

Les sphérules en dispersion ont un diamètre compris entre 0,05µm et 5µm.

La phase aqueuse encapsulée dans les sphérules peut être de l'eau ou une solution aqueuse de substance active et est dans ce cas de préférence isoosmotique par rapport à la phase continue de la dispersion.

Les sphérules peuvent être obtenues en particulier suivant le procédé décrit dans le brevet français 2 315 991 de la demanderesse, selon lequel on prépare une dispersion de sphérules constituées de couches moléculaires organisées renfermant une phase aqueuse à encapsuler, en mettant en contact, d'une part un ou plusieurs composé(s) lipidique(s) de formule (I) associé(s) à un ou plusieurs lipide(s) défini(s) ci-dessus, et d'autre part la phase aqueuse à encapsuler dans les sphérules, en agitant pour assurer le mélange et obtenir une phase lamellaire, en ajoutant ensuite un liquide de dispersion en quantité supérieure à la quantité de phase lamellaire obtenue et en secouant énergiquement pendant une durée allant de 15 minutes à 3 heures environ.

Le rapport pondéral entre la phase aqueuse à encapsuler et le(s) composé(s) de formule (I) associé(s) aux lipides formant la phase lamellaire, est de préférence compris entre 0,1 et 20.

Le rapport pondéral de la phase aqueuse de dispersion que l'on ajoute à la phase lamellaire que l'on disperse, est de préférence compris entre 2 et 100, la phase de dispersion et la phase aqueuse à encapsuler étant de préférence isoosmotiques.

L'agitation est réalisée au moyen d'un agitateur à secousses. Le procédé est de préférence mis en oeuvre à une température comprise entre 30° et 120°C.

Un autre procédé de préparation peut consister à utiliser le procédé dénommé REV (reverse-phase evaporation vesicle) ou évaporation en phase inverse décrit dans Proc. Natl. Acad. Sci. USA., Vol. 75, n° 9, pages 4194-4198 (1978), par SZOKA et PAPAHADJOPOULOS.

On peut également mettre en oeuvre le procédé qui comprend la succession d'étapes consistant à dissoudre au moins un lipide dans au moins un solvant organique non miscible à l'eau; ajouter la phase organique ainsi obtenue à une phase aqueuse; former une dispersion des deux phases sous forte agitation, la taille des vésicules pouvant être réglée en faisant varier la vitesse d'agitation au cours de ce mélange des phases; conduire l'évaporation du (ou des) solvant(s) sous forte agitation; et, le cas échéant, concentrer la dispersion.

Les substances actives peuvent être des substances ayant un intérêt pharmaceutique, alimentaire ou des substances ayant une activité cosmétique. Lorsqu'elles sont hydrosolubles, elles sont dans la phase aqueuse encapsulée à l'intérieur des vésicules.

Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels; des agents de brunissage artificiel tels que la dihydroxyacétone, l'érythrulose, le glycéraldéhyde, les γ-dialdéhydes tels que l'aldéhyde tartrique, ces composés étant éventuellement associés à des colorants; des filtres solaires hydrosolubles; des antiperspirants, des déodorants, des astringents, des produits rafraîchissants, toniques, cicatrisants, kératolytiques, dépilatoires, des eaux parfumées; des extraits de tissus végétaux, tels que les polysaccharides; des colorants hydrosolubles; des agents antipelliculaires; des agents antiséborrhéiques, des oxydants tels que des agents de décoloration comme l'eau oxygénée; des réducteurs tels que l'acide thioglycolique et ses sels.

On peut citer également les vitamines, les hormones, les enzymes telles que la superoxyde dismutase, les vaccins, les anti-inflammatoires tels que l'hydrocortisone, les antibiotiques, les bactéricides, les agents cytotoxiques ou anti-tumoraux.

Lorsque les substances actives sont liposolubles, elles se trouvent incorporées dans les feuillets des vésicules. Elles peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

On peut également ajouter à la phase aqueuse des dispersions de sphérules selon l'invention une phase liquide L non miscible à l'eau. En particulier, la composition selon l'invention peut contenir de 2 à 70% en poids de phase liquide L, non miscible à l'eau, par rapport au poids total de la composition, la proportion pondérale relative de(s) lipide(s) constitutif(s) de vésicules par rapport à la phase liquide dispersée L étant comprise entre 0,02/1 et 10/1.

Le(s) constituant(s) de la phase liquide L dispersée dans la phase aqueuse D, peut (peuvent) être choisi(s) dans le groupe formé par les huiles, telles que les esters d'acides gras et de polyols, et les esters d'acide gras et d'alcools ramifiés de formule R⁸-COOR⁹, formule dans laquelle R⁸ représente le reste d'un acide gras supérieur comportant de 7 à 19 atomes de carbone et R⁹ représente une chaîne hydrocarbonée ramifiée contenant de 3 à 20 atomes de carbone; les hydrocarbures, tels que l'hexadécane, l'huile de paraffine, le perhydrosqualène; les hydrocarbures halogénés, tels que le perfluorodécahydronaphtalène; la perfluorotributylamine; les polysiloxanes; les esters d'acides organiques, les éthers et polyéthers. La phase liquide L peut renfermer au moins un parfum et/ou au moins une substance active liposoluble. De telles substances liposolubles peuvent être constituées par les filtres solaires liposolubles, les substances destinées à améliorer l'état des peaux sèches ou séniles, les tocophérols, les vitamines E ou F, la vitamine A et ses esters, l'acide rétinoïque, les antioxydants, les acides gras essentiels, l'acide glycyrrhétinique, les agents kératolytiques et les caroténoïdes.

On peut également ajouter aux dispersions de sphérules selon l'invention divers adjuvants tels que des opacifiants, des gélifiants, des arômes, des parfums ou des colorants.

Les dispersions de sphérules lipidiques selon l'invention présentent l'intérêt de véhiculer des substances actives qui se trouvent ainsi masquées et protégées vis-à-vis des différents agents d'altération: oxydants et plus généralement les composés réactifs vis-à-vis des substances actives encapsulées. La pénétration et la fixation des substances actives peuvent être modulées par la variation de la taille des sphérules et de leur charge électrique. L'action de ces substances actives peut également être ainsi différée (effet retard). Enfin, il est possible d'obtenir grâce à l'utilisation des lipides (I) selon l'invention, et de substances actives combinées une action bénéfique spécifique de la substance active utilisée et en même temps assouplissante, particulièrement intéressante dans le cas du traitement de la peau.

La présente invention a donc également pour objet l'utilisation en cosmétique d'une dispersion aqueuse de sphérules constituée de couches moléculaires organisées de composés lipidiques (I) associés à d'autres lipides renfermant une phase aqueuse à encapsuler, en particulier pour le traitement de la peau.

L'invention a également pour objet l'utilisation d'une telle dispersion de sphérules lipidiques en dermopharmacie ou dans l'industrie alimentaire.

La présente invention sera mieux illustrée par les exemples non limitatifs suivants.

### EXEMPLE 1

### Préparation du 2-(2'-hydroxy hexadécanoyl)amino octadécane-1,3-diol

### 1ère étape : Préparation du composé (II) avec : R₁ = C₁₅ H₃₁ ; chlorhydrate du 2-amino-1,3-octadécanediol (mélange érythro-thréo).

Le 2-acétamido-3-oxo octadécanoate de méthyle (100 g, soit 0,27M) est mis en suspension dans 1 litre d'éthanol absolu. La température du milieu réactionnel est amenée en dessous de 0°C. A cettte température, on ajoute en trois fois 30,7 g (0,8 M) de borohydrure de sodium et on maintient l'agitation à cette température pendant 3 heures. Le milieu réactionnel est alors porté à reflux du solvant pendant 3 heures. Après refroidissement à la température ordinaire, on ajoute 140 cm³ d'acide chlorhydrique concentré et on porte à nouveau le milieu réactionnel à reflux pendant 3 heures. Ce milieu est filtré encore chaud sur un verre fritté. Le filtrat est concentré à sec sous pression réduite.

Le solide obtenu est recristallisé dans 300 cm³ de mélange de solvant heptane : acétate d'éthyle = 90 : 10. On isole 88 g d'un solide blanc dont l'indice d'acide mesuré dans l'éthanol par une solution de soude N/10 est de 2,99 meq/g.

Le spectre RMN¹³C de ce solide est conforme à la structure attendue.

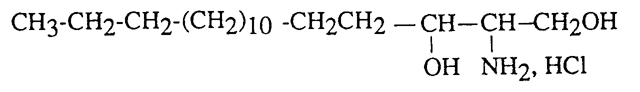

Il s'agit bien du chlorhydrate de sphinganine sous forme de mélange racémique D,L érythro-thréo.

### 2ème étape : Préparation du composé (1) dans lequel -COR₂ est un groupement 2-hydroxy hexadécanoyle.

On solubilise à 65°C, 100 g d'acide 2-hydroxyhexadécanoïque (mélange D,L) dans 800 ml d'acétate d'éthyle. On ajoute 41 g de N-hydroxysuccinimide et laisse revenir la température vers 30°C. On solubilise 83 g de dicyclohexylcarbodiimide dans 200 ml d'acétate d'éthyle, puis on additionne cette solution au milieu réactionnel en 20 minutes. On laisse agiter pendant 5 heures à température ambiante. On filtre les sels précipités sur verre fritté n° 3 et on rince avec 100 ml d'acétate d'éthyle à 40°C. On évapore le filtrat à sec, qui est repris dans 200 ml de tétrahydrofurane. Cette solution, maintenue à 60°C, est ajoutée en 30 minutes dans une solution obtenue par solubilisation à reflux de 108 g de 2-amino-octadécanediol dans 800 ml de tétrahydrofurane et 3 ml de triéthylamine. On laisse sous agitation pendant 2 heures à 60°C. On ajoute 16 ml d'eau et on laisse une nuit à température ambiante. L'insoluble formé est filtré sur verre fritté n° 3. puis lavé par 4 fois 100 ml de tétrahydrofurane à 40°C.

L'ensemble des filtrats est versé dans 6 litres d'eau et 1 ml d'acide chlorhydrique concentré, sous agitation. Le précipité est filtré sur verre fritté n° 1, puis rincé par 300 ml d'acétone. Le produit est séché à l'étuve. Le dérivé est mis en suspension dans 700 ml de dichlorométhane, puis filtré sur verre fritté n° 3 et séché sous vide. On obtient ainsi 130 g (65 % du dérivé brut souhaité), qui est remis en suspension dans 350 ml de pyridine. On ajoute 90 ml d'anhydride acétique en une seule fois. Quand tout est solubilisé, on ajoute 60 ml d'anhydride acétique. On laisse sous agitation pendant 4 heures, à température ambiante. Le milieu réactionnel est précipité dans un mélange constitué de 2,3 litres de méthanol et de 1 litre d'eau, à +4°C, sous agitation. Le précipité est filtré puis rincé par 200 ml d'eau et essoré. Le précipité est ensuite repris par 1 litre d'heptane chaud puis séché sur sulfate de sodium et filtré. On ajoute au filtrat 130 g de silice et on agite 30 minutes. On filtre, la silice est lavée par deux fois 100 ml d'heptane chaud. Les filtrats sont rassemblés et évaporés à sec. On obtient ainsi 150 g brut du dérivé peracétylé qui sont ensuite solubilisés dans 1 litre de méthanol en chauffant légèrement. On ajoute 3,7 g d'une solution méthanolique à 30% de méthylate de sodium et on laisse sous agitation pendant 2 heures. On neutralise avec 24 ml d'un mélange de 22 ml d'eau et 2 ml d'acide chlorhydrique concentré. Le précipité formé est filtré puis séché.

On obtient 98 g de produit solide qui est resolubilisé à 60°C, dans 1 litre d'acétate d'éthyle et qui recristallise à +4°C, pendant 16 heures. Après filtration et séchage, on obtient 93 g (47%) de produit pur.

| **ANALYSE ELEMENTAIRE:** | | | | |
|---|---|---|---|---|
| | % **C** | % **H** | % **N** | % **O** |
| *Théorique* | 73,46 | 12,51 | 2,52 | 11,81 |
| *Trouvé* | 73,56 | 12,55 | 2,53 | 11,68 |
| *Point de fusion* = *89°C* *Rapport érytho/thréo de la chaîne aminodiol 55/45* *Spectre R.M.N.*^{*13*}*C conforme à la structure attendue* | | | | |

### EXEMPLE 2

### Préparation du 2-(2'-hydroxydocosanoyl)amino octadécane-1,3-diol.

On solubilise à 65°C, 50 g d'acide 2-hydroxy-docosanoïque (mélange D/L) dans 400 ml de tétrahydrofurane. On ajoute 15,6 g de N-hydroxysuccinimide. On solubilise 31,9 g de dicyclohexylcarbodiimide dans 100 ml de tétrahydrofurane, puis, lorsque tout est solubilisé, on additionne lentement cette solution au milieu réactionnel en 20 minutes.

On laisse agiter pendant 2 h 30. On filtre les sels sur verre fritté n°3 qui sont rincés avec 50 ml de tétrahydrofurane. Cette solution, maintenue à 60°C, est additionnée en 30 minutes dans une solution obtenue par solubilisation à reflux de 41,4 g de 2-amino-octadécane-1,3-diol (obtenu à l'étape 1 de l'exemple 1) dans 400 ml de tétrahydrofurane et 1,5 ml de triéthylamine. On laisse agiter à 60°C pendant 2 heures. On ajoute 16 ml d'eau et on laisse une nuit à température ambiante.
Le produit insoluble formé est filtré sur verre fritté n° 3, puis lavé par 100 ml de tétrahydrofurane à 45°C.
Le filtrat est versé sous agitation, en 20 minutes, dans 3,5 litres d'eau et 0,5 ml d'acide chlorhydrique concentré. Le précipité obtenu est filtré sur verrre fritté n° 1, essoré, rincé par 300 ml d'acétone, puis séché. Le produit obtenu est mis en suspension dans 200 ml de dichlorométhane, filtré sur verrre fritté n° 3 et essoré. On obtient ainsi 75 g de dérivé brut qui sont remis en suspension dans 180 ml de pyridine. On ajoute 50 ml d'anhydride acétique en une seule fois. Quand tout est solubilisé, on ajoute 50 ml d'anhydride acétique. On laisse sous agitation pendant quatre heures à température ambiante. Le milieu réactionnel est précipité dans un mélange constitué de 2 litres de méthanol et de 0,5 litre d'eau, à +4°C, sous agitation. Le précipité formé est filtré puis rincé par 200 ml d'eau et essoré. Le précipité est repris par 600 ml d'heptane chaud et séché sur sulfate de sodium et filtré. On ajoute au filtrat 80 g de silice et on agite 45 minutes. On filtre, et la silice est lavée par deux fois 60 ml de dichlorométhane. Les filtrats sont rassemblés et évaporés à sec. On obtient 81 g brut de dérivé peracétylé qui sont ensuite solubilisés dans 55 ml de dichlorométhane et 770 ml de méthanol. On ajoute 1,78 g d'une solution méthanolique à 30% de méthylate de sodium et on laisse sous agitation pendant 3 heures. On neutralise avec 12 ml d'un mélange constitué de 11 ml d'eau et de 1 ml d'acide chlorhydrique concentré. Le précipité formé est filtré puis séché. On obtient ainsi 53 g de produit solide qui sont resolubilisés dans 530 ml d'acétate d'éthyle à 60°C et qui recristallisent à +4°C en une nuit. Après filtration et séchage, on obtient 48 g (71%) de produit pur.

| **ANALYSE ELEMENTAIRE:** | | | | |
|---|---|---|---|---|
| | % **C** | % **H** | % **N** | % **O** |
| *Théorique* | 75,06 | 12,76 | 2,19 | 10 |
| *Trouvé* | 75,05 | 12,77 | 2,11 | 10,3 |
| *Point de fusion* = *93°C* *Rapport érythro/thréo du 2-aminooctadécane-1,3 diol : 54/46* *Spectre RMN*^{*13*}*C conforme à la structure attendue.* | | | | |

### EXEMPLE 3

### Préparation du 2-(D,L-mandéloyl)amino octadécane-1,3-diol.

On solubilise 10 g d'acide mandélique D,L dans 50 ml de tétrahydrofurane, puis on ajoute 7,6 g de N-hydroxysuccinimide. On solubilise 14,95 g de dicyclohexylcarbodiimide dans 50 ml de tétrahydroflurane, puis on additionne cette solution au milieu réactionnel. On laisse agiter pendant 3 heures à température ambiante, puis les sels formés sont filtrés sur verre fritté n° 3 et on rince avec 50 ml de tétrahydrofurane.

Cette solution, maintenue à 60°C, est additionnée en 30 minutes dans une solution obtenue par solubilisation à reflux de 18,8 g de 2-amino-octadécane-1,3-diol (obtenu à l'étape 1 de l'exemple 1) dans 100 ml de tétrahydrofurane.

On laisse sous agitation à 60°C pendant 3 heures, puis on verse le mélange dans de l'eau.

Le précipité formé est filtré, lavé et séché sous vide à 40°C.
Le produit brut obtenu est recristallisé dans 250 ml d'acétate de méthyle.

On obtient ainsi 20 g de produit pur (Rendement 74 %).

| **ANALYSE ELEMENTAIRE** | | | | |
|---|---|---|---|---|
| | % **C** | % **H** | % **N** | % **O** |
| *Théorique* | 71,68 | 10,41 | 3,22 | 14,69 |
| *Trouvé* | 71,7 | 10,48 | 3,29 | 14,89 |
| *Point de fusion : 98-115°C* *Spectre RMN13C conforme à la structure attendue* *Spectre de Masse conforme à la structure attendue* *Rapport érythro/thréo de la chaîne aminodiol : 61/39* | | | | |

### Exemple A

### Fond de teint protecteur

On a préparé un fond de teint ayant la composition suivante :

| | |
|---|---|
| - Silicate d'aluminium et de magnésium | 0,50 g |
| - Carboxyméthyl cellulose vendu sous la dénomination "BLANOSE 7 LF" par la Société AQUALON | 0,15 g |
| - Glycérine | 3,00 g |
| - Conservateurs qs | |
| - 2-(2'-hydroxyhexadécanoyl)amino octadécane- 1,3-diol (composé de l'exemple 1) | 1,00 g |
| - Alcool de lanoline | 1,50 g |
| - Stéarate de glycérol | 1,00 g |
| - Acide stéarique | 2,50 g |
| - Triéthanolamine | 1,50 g |
| - Triglycérides de l'acide caprique/caprilique vendus sous la dénomination "MIGLYOL 812" par la Société HÜLS | 6,00 g |
| - Squalane | 10,00 g |
| - Poudre de polyéthylène | 3,00 g |
| - Pigments | 10,00 g |
| - Eau déminéralisée qsp | 100 g |

Le fond de teint obtenu a un effet hydratant amélioré.

### Exemple B

### Rouge à lèvres traitant

On a préparé un rouge à lèvres ayant la composition suivante :

| | |
|---|---|
| - Lanoline liquide | 17,50 g |
| - Cire microcristalline | 15,00 g |
| - Triglycérides d'acides caprique/caprilique vendus sous la dénomination "MIGLYOL 812" par la Société HÜLS | 11,00 g |
| - Béhénate d'octyl glycéryle | 11,0 g |
| - 2-(2'-hydroxy-hexadécanoyl)-amino octadécane-1,3-diol (composé de l'exemple 1) | 0,20 g |
| - Micatitane | 10,00 g |
| - Pigments organiques | 8,00 g |
| - Huile de ricin qsp | 100 g |

Le rouge à lèvres obtenu a un effet hydratant amélioré.

### Exemple C

### Mascara-crème

On a préparé un mascara-crème ayant la composition suivante :

| | |
|---|---|
| - Stéarate de triéthanolamine | 10,0 g |
| - Cire de candellila | 15,0 g |
| - Cire d'abeille | 17,0 g |
| - Gomme de xanthane | 1,0 g |
| - 2-(2'-hydroxy-hexadécanoyl)amino ocadécane-1,3-diol (composé de l'exemple 1) | 0,5 g |
| - Oxyde de fer noir | 5,0 g |
| - Polysulfure d'aminosilicate | 4,0 g |
| - Conservateurs qs | |
| - Eau qsp | 100 g |

Le mascara-crème obtenu a un effet hydratant amélioré.

### Exemple D

### Crème protectrice pour les mains

On a préparé une crème protectrice pour les mains ayant la composition suivante :

| | |
|---|---|
| - Stéarate de sorbitane polyoxyéthyléné à 20 moles d'oxyde d'éthylène vendu sous la dénomination "TWEEN 60" par la Société ICI | 2,0 g |
| - Alcool cétylique | 1,0 g |
| - Huile de silicone de viscosité 200 centistokes (2.10⁻⁴ m²/s) | 7,0 g |
| - Propylène glycol | 2,0 g |
| - Acide polyacrylique réticulé vendu sous la dénomination "CARBOPOL 940" par la Société GOODRICH | 0,3 g |
| - Parahydroxybenzoate de méthyle | 0,3 g |
| - 2-(2'-hydroxyhexadécanoyl) amino octadécane-1,3-diol (composé de l'exemple 1) | 0,5 g |
| - Eau déminéralisée qsp | 100 g |

La crème protectrice obtenue a un effet hydratant amélioré.

### Exemple E

### Crème de soin E/H teintée

On a préparé une crème de soin ayant la composition suivante :

| | |
|---|---|
| - Céthyldiméthicone copolyol vendu sous la dénomination "ABIL EM 90" par la Société GOLDSCHMIDT | 5,0 g |
| - Stéaralkonium hectorite | 2,0 g |
| - Octyldodécanol | 8,0 g |
| - Décaméthylcyclopentasiloxane | 20,0 g |
| - Parfum qs | |
| - Conservateur qs | |
| - Glycérine | 3,0 g |
| - Oxyde de titane enrobé de silicone vendu sous la dénomination "Cosmétic White SIC" par la Société MIYOSHI KASEI | 4,9 g |
| - Oxyde de fer noir enrobé de silicone vendu sous la dénomination "Cosmetic black SIC" par la Société MIYOSHI KASEI | 0,7 g |
| - Oxyde de fer jaune enrobé de silicone vendu sous la dénomination "Cosmetic Yellow SIC" par la Société MIYOSHI KASEI | 0,7 g |
| - Oxyde de fer rouge enrobé de silicone vendu sous la dénomination "Cosmetic Russet SIC" par la Société MIYOSHI KASEI | 0,7 g |
| - Composé de l'exemple 2 | 0,50 g |
| - Chlorure de sodium | 1,50 g |
| - Eau déminéralisée qsp | 100 g |

La crème obtenue a un effet hydratant amélioré.

### Exemple F

### Crème de jour protectrice

On a préparé une crème de jour protectrice ayant la composition suivante :

| | |
|---|---|
| - Stéarate de glycérol auto-émulsionnable vendu sous la dénomination "ARLACEL 165" par la Société ICI | 3,0 g |
| - Alcool cétylique | 0,5 g |
| - Alcool stéarylique | 0,5 g |
| - Squalane | 15,0 g |
| - Huile de sésame | 10,0 g |
| - Acide stéarique | 3,0 g |
| - 2-hydroxy 4-méthoxybenzophénone vendu sous la dénomination "UVINUL M40" par la Société BASF | 1,0 g |
| - Glycérine | 5,0 g |
| - Parahydroxybenzoate de méthyle | 0,3 g |
| - Composé de l'exemple 2 | 0,3 g |
| - Eau déminéralisée qsp | 100 g |

La crème obtenue a un effet hydratant amélioré.

### Exemple G

### Mascara

On a préparé un mascara ayant la composition suivante :

| | |
|---|---|
| - Stéarate de triéthanolamine | 15,0 g |
| - Paraffine | 3,0 g |
| - Cire d'abeille | 8,0 g |
| - Composé de l'exemple 2 | 0,5 g |
| - Colophane | 2,0 g |
| - Ozokérite | 10,0 g |
| - Conservateurs qs | |
| - Gomme arabique | 0,5 g |
| - Hydrolysat de kératine vendu sous la dénomination "KERAZOL" par la Société CRODA | 1,0 g |
| - Pigments | 6,0 g |
| - Eau déminéralisée qsp | 100 g |

Le mascara obtenu a un effet hydratant amélioré.

### Exemple H

### Base traitante pour ongle

On a préparé une base traitante pour ongle ayant la composition suivante :

| | |
|---|---|
| - Nitrocellulose | 12,0 g |
| - Résine toluène sulfonamide-formaldéhyde | 9,0 g |
| - Camphre | 1,0 g |
| - Phtalate de dibutyle | 6,05 g |
| - Acétate de butyle | 24,0 g |
| - Acétate d'éthyle | 9,05 g |
| - Alcool isopropylique | 6,0 g |
| - Stéaralkonium hectorite | 1,0 g |
| - Composé de l'exemple 2 | 0,01 g |
| - Acide citrique | 0,02 g |
| - Toluène qsp | 100 g |

La base traitante obtenue a un effet hydratant amélioré.

### Exemple I

### Rouge à lèvres

On a préparé un rouge à lèvres ayant la composition suivante :

| | |
|---|---|
| - Parfum | 0,50 g |
| - Triglycérides capryliques/capriques vendus sous la dénomination "MIGLYOL 812" par la Société HÜLS | 9,10 g |
| - Huile de ricin | 9,10 g |
| - Butylhydroxytoluène | 0,16 g |
| - Lanoline liquide | 12,80 g |
| - Lanoline isopropyle | 4,50 g |
| - Composé de l'exemple 2 | 0,50 g |
| - Cire microcristalline | 11,0 g |
| - Copolymère d'acétate de vinyle/stéarate d'allyle dans un rapport 65/35 | 4,50 g |
| - Béhénate d'octyl glycéryle | 9,10 g |
| - Pigments | 9,00 g |
| - Huile de sésame qsp | 100 g |

Le rouge à lèvres obtenu a un effet hydratant amélioré.

### Exemple J

### Crème de jour pour le visage

### Première phase

| Phase lipidique | |
|---|---|
| - Composé de l'exemple 1 | 1,0 g |
| - Palmitate de sorbitane | 1,0 g |
| - Cholestérol | 0,7 g |
| - Acylglutamate de sodium vendu sous la dénomination "HS 11" par la Société AJINOMOTO | 0,3 g |
| - Acétate d'α tocophérol | 0,3 g |

| Phase aqueuse | |
|---|---|
| - Glycérine | 3,0 g |
| - Conservateurs | 0,4 g |
| - Acide citrique | 0,02 g |
| - Eau déminéralisée qsp | 50 g |

### Deuxième phase

| | |
|---|---|
| - Huile de silicone volatile | 10,0 g |
| - Huile d'amandes d'abricots | 10,0 g |
| - Parfum | 0,2 g |
| - Mélange d'acides carboxyvinyliques vendu sous la dénomination "CARBOPOL 940" par la Société GOODRICH | 0,42 g |
| - Triéthanolamine qs pH 6,5 | |
| - Eau déminéralisée qsp | 100 g |

Dans une première phase, on prépare la phase lipidique par mélange sous forme liquide des différents lipides amphiphiles la composant, et associés à l'acétate d'α-tocophérol. On met la phase lipidique obtenue en présence de la phase aqueuse, de façon à obtenir une phase lamellaire.

On ajoute à la phase lamellaire hydratée obtenue la deuxième phase. On soumet le mélange à une agitation énergique dans un homogénéiseur pour obtenir des vésicules dispersées dans une phase aqueuse de dispersion.

On obtient ainsi une crème blanche, de viscosité égale à 15 poises (1,5 Pa/s). La crème obtenue a un effet hydratant amélioré.

Le composé de l'exemple 1 peut être remplacé par le composé de l'exemple 2.

## Revendications

1. Composé caractérisé en ce qu'il répond à la formule : 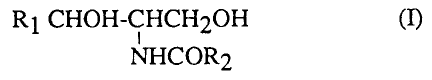 dans laquelle :
R₁ est un radical alkyle ou alcényle en C₁₁ à C₂₁, de préférence en C₁₃ à C₁₉ ;
R₂ est un radical hydroxyalkyle linéaire ou ramifié, en C₁ à C₂₉, de préférence linéaire en C₁ à C₂₁, ou un radical hydroxyaralkyle en C₇ à C₂₉, de préférence en C₇ à C₁₉, le groupement hydroxy étant en position alpha du carbonyle, le composé étant sous forme d'un mélange racémique des diastéréoisomères érythro et thréo, pour la partie aminodiol 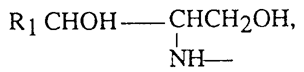 dans les proportions érythro : thréo de 85 : 15 à 20 : 80, de préférence de 65:35 à 45:55.

2. Composé selon la revendication 1 caractérisé en ce que R₁ est le radical pentadécyle.

3. Composé selon la revendication 1 caractérisé en ce que le radical R₂ est choisi parmi les radicaux 1-hydroxy pentadécyle, 1-hydroxyhénéicosyle et D,L 1-hydroxy benzyle.

4. Composé selon la revendication 1, caractérisé en ce qu'il est choisi parmi le 2-(2'-hydroxyhexadécanoyl)amino octadécane-1,3-diol, le 2-(2'-hydroxydocosanoyl)amino octadécane-1,3-diol et le 2-(D,L-mandéloyl)amino octadécane-1,3-diol.

5. Procédé de préparation d'un composé de formule (I) selon la revendication 1, caractérisé en ce qu'il comprend :
(**a**) l'acylation en milieu anhydre ou en présence d'un solvant approprié, de la fonction amine d'une sphingosine ou d'une sphinganine de formule : 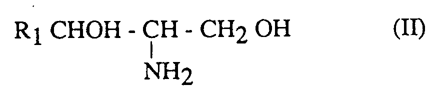 dans laquelle R₁ est un radical alkyle ou alcènyle en C₁₁ à C₂₁, ce composé étant un racémique des diastérioisomères érythro et thréo dans les proportions érythro-thréo de 85 : 15 à 20 : 80, de préférence de 65:35 à 45:55, à l'aide d'un agent acylant choisi parmi les chlorures d'acide, les anhydrides d'acide, les anhydrides mixtes, les esters de paranitrophénol, les esters de succinimide, les esters de carbodiimide, les esters d'alkyle inférieur en C₁ à C₈, les azolides et les O-carboxyanhydrides des 2-hydroxy acides correspondants, pour obtenir un composé de formule : 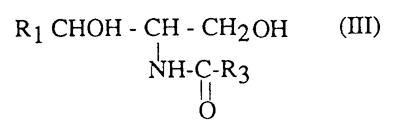 dans laquelle R₁ est défini comme précédemment et R₃ représente soit le radical R₂ soit un radical choisi parmi les radicaux alkyles linéaires ou ramifiés en C₁ à C₂₉, de préférence linéaires en C₁ à C₂₁, ou aralkyles en C₇ à C₂₉, de préférence en C₇ à C₁₉, substitués en position α par rapport au carbonyle, le substituant étant choisi parmi les radicaux -Br, -Cl, -I et -OB, -OB étant un groupe susceptible de former un groupe -OH;
(**b**) l'isolation du composé de formule (III); et
(**c**) éventuellement, lorsque R₃ est différent de R₂, l'hydrolyse du composé de formule (III) obtenu pour transformer le groupe -OB en groupe hydroxyle.

6. Procédé selon la revendication 5, caractérisé en ce qu'il comprend après l'étape (a) d'acylation de la fonction amine de la sphingosine ou de la sphinganine de formule (II) et avant l'étape (b) d'isolation du composé de formule (III), une étape de protection des groupements hydroxyle consistant à faire réagir dans le mélange résultant de l'étape (a) un agent protecteur des groupes -OH choisi parmi les anhydrides d'acide, les halogénures d'acide et les chlorosilanes; et après l'étape (b) d'isolation du composé de formule (III), l'hydrolyse du composé de formule (III), de préférence en milieu basique; et la récupération du composé de formule (I).

7. Procédé selon la revendication 6, caractérisé en ce que l'agent protecteur des groupes hydroxyle est choisi parmi l'anhydride acétique, le chlorure d'acétyle, le chlorure de benzoyle, le chlorure de benzyle, le bromure de benzyle et les chlorosilanes de formules ClSi(CH₃)₃, ClSi(CH₃)₂(tBu), et ClSi(tBu)(-C₆H₅)₂.

8. Procédé selon l'une quelconque des revendications 5 à 7 caractérisé en ce que l'agent acylant répond à la formule R₃ COA (IV)
dans laquelle R₃ représente :
- soit le radical R₂,
- soit un radical choisi parmi les radicaux alkyles linéaires ou ramifiés en C₁ à C₂₉, de préférence linéaires en C₁ à C₂₁, et aralkyles en C₇ à C₂₉, de préférence en C₇ à C₁₉, substitués en position α par rapport au carbonyle, le substituant en position α par rapport au carbonyle étant choisi parmi les radicaux -Br, -Cl, -I et -OB, -OB étant un groupe susceptible de former un groupe -OH;
A est choisi parmi les halogènes, 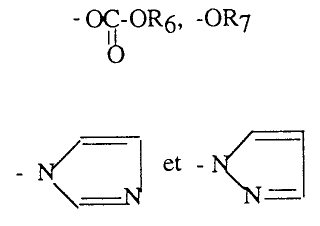 où R₆ est un radical alkyle inférieur en C₂ à C₈ et R₇ est choisi parmi les radicaux alkyles inférieurs en C₁ à C₈, 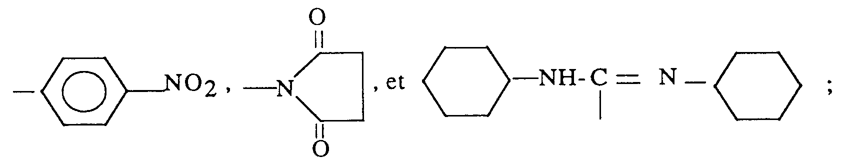 à la condition que lorsque R₃ représente le radical R₂, A soit différent de -Cl.

9. Procédé selon la revendication 8 caractérisé en ce que le radical -OB de l'agent acylant est choisi parmi les groupements acétate, benzoate, benzyloxy, OSi(CH₃)₃, -OSi(CH₃)₂(tBu), -OSi(tBu) (-C₆H₅)₂.

10. Procédé selon la revendication 8 caractérisé en ce que l'agent acylant est choisi parmi le 2-hydroxy hexadécanoate de succinimide, le 2-hydroxyhexadécanoate de dicyclohexylcarbodiimide, le 2-hydroxy docosanoate de succinimide, le 2-hydroxydocosanoate de dicyclohexylcarbodiimide et le D, L mandéloate de succinimide.

11. Procédé selon la revendication 10, caractérisé en ce que le composé de formule (II) est le 2-amino-octadécane-1,3-diol.

12. Composition à usage cosmétique ou dermopharmaceutique, caractérisée par le fait qu'elle contient un composé de formule (I) selon l'une quelconque des revendications 1 à 4.

13. Composition selon la revendication 12, caractérisée par le fait qu'elle contient 0,05 à 20 % en poids, de préférence 0,1 à 10 % en poids, de composé de formule (I).

14. Composition selon la revendication 12 ou 13 caractérisée en ce qu'elle comprend en outre un adjuvant choisi parmi les corps gras, les solvants, l'eau, les épaississants, les émulsionnants, les produits hydratants, les adoucissants, les filtres solaires, les germicides, les colorants, les conservateurs, les parfums, les propulseurs et les tensio-actifs.

15. Composition selon l'une quelconque des revendications 12 à 14, caractérisée par le fait qu'elle se présente sous forme d'émulsion dont la phase grasse, représentant 5 à 60 % du poids total de l'émulsion, est essentiellement constituée d'un mélange de composé de formule (I) avec au moins une huile, la phase aqueuse constituant 30 à 85 % du poids total de l'émulsion, l'agent émulsionnant étant présent à raison de 1 à 20 % en poids, et de préférence 2 à 12 % en poids par rapport au poids total de l'émulsion.

16. Composition selon l'une quelconque des revendications 12 à 14, caractérisée par le fait qu'elle se présente sous forme de lotion huileuse, oléoalcoolique ou hydroalcoolique, sous forme de gel, de dispersion ou de bâtonnets solides, de spray ou de mousse aérosol.

17. Composition selon l'une quelconque des revendications 12 à 14, caractérisée par le fait qu'elle se présente sous forme d'une dispersion aqueuse de sphérules lipidiques, constituées de couches moléculaires organisées renfermant une phase aqueuse encapsulée, ces couches étant constituées d'au moins un composé de formule (I) associé à au moins un autre composé lipidique.

18. Composition sous forme de dispersion aqueuse de sphérules lipidiques selon la revendication 17, caractérisée par le fait que l'autre composé lipidique est choisi parmi les alcools et diols à longue chaîne, les stérols, les phospholipides, les glycolipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras, les alkylsulfates et les acides gras sous forme de sels.

19. Composition sous forme de dispersion aqueuse de sphérules lipidiques selon la revendication 17 ou 18, caractérisée par le fait que les sphérules ont un diamètre compris entre 0,05 µm et 5 µm.

20. Composition sous forme de dispersion aqueuse de sphérules lipidiques selon l'une quelconque des revendications 17 à 19, caractérisée par le fait que la phase aqueuse encapsulée dans les sphérules est de l'eau ou une solution aqueuse de substance active.

21. Composition sous forme de dispersion aqueuse de sphérules lipidiques selon l'une quelconque des revendications 17 à 20, caractérisée par le fait que la phase aqueuse encapsulée dans les sphérules contient au moins une substance active hydrosoluble cosmétique et/ou pharmaceutique choisie parmi les humectants, les agents de brunissage artificiel éventuellement associés à des colorants, les filtres solaires hydrosolubles, les antiperspirants, les déodorants, les astringents, les produits rafraîchissants, les toniques, les cicatrisants, les kératolytiques, les dépilatoires, les eaux parfumées, les extraits de tissus végétaux, les colorants hydrosolubles, les agents anti-pelliculaires, les agents-séborrhéiques, les oxydants, les réducteurs, les vitamines, les hormones, les enzymes, les vaccins, les anti-inflammatoires, les les antibiotiques, les bactéricides et les agents cytotoxiques ou antitumoraux.

22. Composition sous forme de dispersion aqueuse de sphérules lipidiques selon l'une quelconque des revendications 17 à 20, caractérisée par le fait qu'elle contient au moins une substance active liposoluble choisie parmi les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

23. Composition sous forme de dispersion aqueuse de sphérules lipidiques selon l'une quelconque des revendications 17 à 22, caractérisée par le fait qu'elle comprend 2 à 70 % en poids d'une phase liquide non miscible à l'eau choisie parmi les huiles, les hydrocarbures, les hydrocarbures halogénés, la perfluorotributylamine, les polysiloxanes, les esters d'acides organiques, les éthers et polyéthers.

24. Utilisation en cosmétique d'un composé de formule (I) selon l'une quelconque des revendications 1 à 4.

25. Utilisation du composé de formule (I) selon l'une quelconque des revendications 1 à 4, en tant que constituant cireux ayant des propriétés émollientes et adoucissantes dans des émulsions, des dispersions, des gels, des bâtonnets solides ou dans des lotions à usage cosmétique.

26. Utilisation du composé de formule (I) selon l'une quelconque des revendications 1 à 4 en association avec au moins un autre composé lipidique, pour la formation de dispersions de sphérules lipidiques à usage cosmétique.

27. Procédé de traitement cosmétique hydratant de la peau, des cheveux ou des poils, caractérisé par le fait qu'il consiste à appliquer sur la peau, les cheveux ou les poils une quantité suffisante d'une composition selon l'une quelconque des revendications 12 à 23.

## Claims

1. Compound characterized in that it corresponds to the formula: 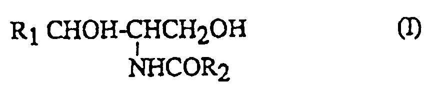 in which:
R₁ is a C₁₁ to C₂₁, preferably C₁₃ to C₁₉, alkyl or alkenyl radical;
R₂ is a linear or branched C₁ to C₂₉, preferably linear C₁ to C₂₁, hydroxyalkyl radical or a C₇ to C₂₉, preferably C₇ to C₁₉, hydroxyaralkyl radical, the hydroxyl group being in the position alpha to the carbonyl, the compound being in the form of a racemic mixture of the erythro and threo diastereoisomers for the aminodiol part 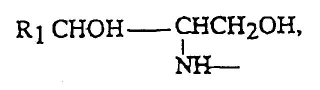 in erythro/threo ratios of from 85 : 15 to 20 : 80, preferably from 65:35 to 45:55.

2. Compound according to Claim 1, characterized in that R₁ is the pentadecyl radical.

3. Compound according to Claim 1, characterized in that the radical R₂ is chosen from the 1-hydroxypentadecyl, 1-hydroxyheneicosyl and D,L-1-hydroxybenzyl radicals.

4. Compound according to Claim 1, characterized in that it is chosen from 2-(2'-hydroxyhexadecanoyl)aminooctadecane-1,3-diol, 2- (2'-hydroxydocosanoyl)aminooctadecane-1,3-diol and 2- (D,L-mandeloyl)aminooctadecane-1,3-diol.

5. Process for the preparation of a compound of formula (I) according to Claim 1, characterized in that it comprises:
(a) acylation in an anhydrous medium, or in the presence of a suitable solvent, of the amine function of a sphingosine or of a sphinganine of formula: 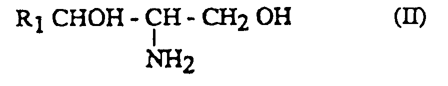 in which R₁ is a C₁₁ to C₂₁ alkyl or alkenyl radical, this compound being a racemic mixture of the erythro and threo diastereoisomers in erythro/threo ratios of from 85 : 15 to 20 : 80, preferably from 65:35 to 45:55, using an acylating agent chosen from acid chlorides, acid anhydrides, mixed anhydrides, para-nitrophenol esters, succinimide esters, carbodiimide esters, C₁ to C₈ lower alkyl esters, azolides and O-carboxy anhydrides of the corresponding 2-hydroxy acids, in order to obtain a compound of formula: 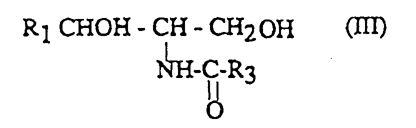 in which R₁ is defined as above and R₃ represents either the radical R₂ or a radical chosen from linear or branched C₁ to C₂₉, preferably linear C₁ to C₂₁, alkyl radicals or C₇ to C₂₉, preferably C₇ to C₁₉, aralkyl radicals which are substituted in the α position relative to the carbonyl, the substituent being chosen from the radicals -Br, -Cl, -I and -OB, -OB being a group capable of forming an -OH group;
(b) isolation of the compound of formula (III); and
(c) optionally, when R₃ is different from R₂, hydrolysis of the compound of formula (III) obtained in order to convert the group -OB into a hydroxyl group.

6. Process according to Claim 5, characterized in that it comprises, after the step (a) for acylation of the amine function of the sphingosine or sphinganine of formula (II) and before the step (b) for isolation of the compound of formula (III), a step for protection of the hydroxyl groups consisting in reacting the mixture resulting from step (a) with a protecting agent for -OH groups chosen from acid anhydrides, acid halides and chlorosilanes; and, after the step (b) for isolation of the compound of formula (III), hydrolysis of the compound of formula (III), preferably in a basic medium; and recovery of the compound of formula (I).

7. Process according to Claim 6, characterized in that the agent for protecting the hydroxyl groups is chosen from acetic anhydride, acetyl chloride, benzoyl chloride, benzyl chloride, benzyl bromide and the chlorosilanes of formulae ClSi(CH₃)₃, ClSi(CH₃)₂(tBu) and ClSi(tBu)(C₆H₅)₂.

8. Process according to any one of Claims 5 to 7, characterized in that the acylating agent corresponds to the formula R₃COA (IV)
in which R₃ represents:
- either the radical R₂
- or a radical chosen from linear or branched C₁ to C₂₉, preferably linear C₁ to C₂₁, alkyl radicals and C₇ to C₂₉, preferably C₇ to C₁₉, aralkyl radicals which are substituted in the α position relative to the carbonyl, the substituent in the α position relative to the carbonyl being chosen from the radicals -Br, -Cl, -I and -OB, -OB being a group capable of forming an -OH group;
A is chosen from halogens, 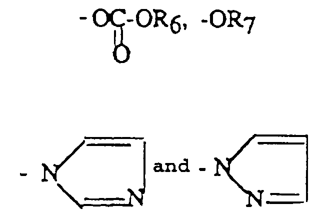 where R₆ is a C₂ to C₈ lower alkyl radical and R₇ is chosen from C₁ to C₈ lower alkyl radicals, 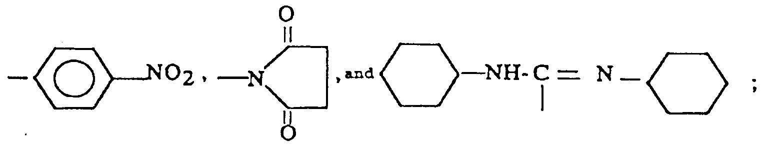 on condition that, when R₃ represents the radical R₂, A is other than -Cl.

9. Process according to Claim 8, characterized in that the radical -OB of the acylating agent is chosen from acetate, benzoate, benzyloxy, -OSi(CH₃)₃, -OSi(CH₃)₂(tBu) and -OSi(tBu)(C₆H₅)₂ groups.

10. Process according to Claim 8, characterized in that the acylating agent is chosen from succinimide 2 -hydroxydecanoate, dicyclohexylcarbodiimide 2-hydroxyhexadecanoate, succinimide 2-hydroxydocosanoate, dicyclohexylcarbodiimide 2-hydroxydocosanoate and succinimide D,L-mandelate.

11. Process according to Claim 10, characterized in that the compound of formula (II) is 2-aminooctadecane-1,3-diol.

12. Composition for cosmetic or dermopharmaceutical use, characterized in that it contains a compound of formula (I) according to any one of Claims 1 to 4.

13. Composition according to Claim 12, characterized in that it contains 0.05 to 20% by weight, preferably 0.1 to 10% by weight, of compound of formula (I).

14. Composition according to Claim 12 or 13, characterized in that it additionally comprises an adjuvant chosen from fatty substances, solvents, water, thickening agents, emulsifying agents, moisturizing products, softeners, sunscreen agents, germicides, dyes, preserving agents, perfumes, propellants and surfactants.

15. Composition according to any one of Claims 12 to 14, characterized in that it is provided in the form of an emulsion the fatty phase of which, representing 5 to 60% of the total weight of the emulsion, consists essentially of a mixture of compound of formula (I) with at least one oil, the aqueous phase constituting 30 to 85% of the total weight of the emulsion, the emulsifying agent being present in an amount of from 1 to 20% by weight and preferably 2 to 12% by weight relative to the total weight of the emulsion.

16. Composition according to any one of Claims 12 to 14, characterized in that it is provided in the form of an oily, oleo-alcoholic or aqueous-alcoholic lotion or in the form of a gel, a solid dispersion or solid sticks, a spray or an aerosol foam.

17. Composition according to any one of Claims 12 to 14, characterized in that it is provided in the form of an aqueous dispersion of lipid spherules, consisting of organized molecular layers containing an encapsulated aqueous phase, these layers being composed of at least one compound of formula (I) associated with at least one other lipid compound.

18. Composition in the form of an aqueous dispersion of lipid spherules according to Claim 17, characterized in that the other lipid compound is chosen from long chain alcohols and diols, sterols, phospholipids, glycolipids, cholesteryl sulphate and phosphate, long chain amines and their quaternary ammonium derivatives, dihydroxyalkylamines, polyoxyethylenated fatty amines, long chain amino alcohol esters, their salts and quaternary ammonium derivaties, phosphoric esters of fatty alcohols, alkyl sulphates and fatty acids in the form of salts.

19. Composition in the form of an aqueous dispersion of lipid spherules according to Claim 17 or 18, characterized in that the spherules have a diameter between 0.05 µm and 5 µm.

20. Composition in the form of an aqueous dispersion of lipid spherules according to any one of Claims 17 to 19, characterized in that the aqueous phase encapsulated in the spherules is water or an aqueous solution of active substance.

21. Composition in the form of an aqueous dispersion of lipid spherules according to any one of Claims 17 to 20, characterized in that the aqueous phase encapsulated in the spherules contains at least one cosmetic and/or pharmaceutical water-soluble active substance chosen from moistening agents, artificial tanning agents possibly associated with dyes, water-soluble sunscreen agents, antiperspirants, deodorants, astringents, freshening, tonic, cicatrizing, keratolytic and depilatory products, perfumed waters, vegetable tissue extracts, water-soluble dyes, anti-dandruff agents, anti-seborrhoeic agents, oxidizing agents, reducing agents, vitamins, hormones, enzymes, vaccines, anti-inflammatory agents, antibiotics, bactericides and cytotoxic or anti-tumour agents.

22. Composition in the form of an aqueous dispersion of lipid spherules according to any one of Claims 17 to 20, characterized in that it contains at least one lipid-soluble active substance chosen from lipid-soluble sunscreen agents, substances intended to improve the condition of dry or senile skin, tocopherols, vitamins E, F or A and their esters, retinoic acid, antioxidants, essential fatty acids, glycyrrhetinic acid, keratolytic agents and carotenoids.

23. Composition in the form of an aqueous dispersion of lipid spherules according to any one of Claims 17 to 22, characterized in that it comprises 2 to 70% by weight of a water-immiscible liquid phase chosen from oils, hydrocarbons, halogenated hydrocarbons, perfluorotributylamine, polysiloxanes, organic acid esters, ethers and polyethers.

24. Use in cosmetics of a compound of formula (I) according to any one of Claims 1 to 4.

25. Use of the compound of formula (I) according to any one of Claims 1 to 4 as a waxy constituent having emollient and softening properties in emulsions, dispersions, gels or solid sticks or in lotions for cosmetic use.

26. Use of the compound of formula (I) according to any one of Claims 1 to 4 in association with at least one other lipid compound, for the formation of dispersions of lipid spherules for cosmetic use.

27. Cosmetic treatment process for moisturizing the skin, head hair or body hair, characterized in that it consists in applying to the skin, head hair or body hair a sufficient amount of a composition according to any one of Claims 12 to 23.

## Patentansprüche

1. Verbindung, dadurch **gekennzeichnet**, daß sie die Formel aufweist: 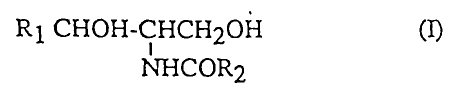 worin gilt:
R₁ ist ein C₁₁₋₂₁- und vorzugsweise ein C₁₃₋₁₉-Alkyl- oder -Alke-nylrest;
R₂ ist ein linearer oder verzweigter C₁₋₂₉ und vorzugsweise ein linearer C₁₋₂₁-Hydroxialkyl- oder ein C₇₋₂₉ und vorzugsweise ein C₇₋₁₉-Hydroxiaralkylrest, worin die Hydroxigruppe in α-Position zur Carbonylgruppe angeordnet ist, und wobei die Verbindung in Form einer razemischen Mischung aus Erythro- und Threo-Diastereoisomeren vorliegt, und zwar für den Aminodiol-Teil: 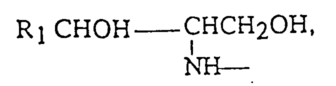 in Erythro-:Threo-Verhältnissen von 85:15 bis 20:80 und vorzugsweise von 65:35 bis 45:55.

2. Verbindung gemäß Anspruch 1,
dadurch **gekennzeichnet**, daß R₁ der Pentadecylrest ist.

3. Verbindung gemäß Anspruch 1,
dadurch **gekennzeichnet**, daß der Rest R₂ aus 1-Hydroxipentadecyl-, 1-Hydroxiheneicosyl- und aus D,L-1-Hydroxibenzylresten ausgewählt ist.

4. Verbindung gemäß Anspruch 1,
dadurch **gekennzeichnet**, daß sie aus 2-(2'-Hydroxihexadecanoyl)aminooctadecan-1,3-diol,2-(2'-Hydroxidocosanoyl)aminooctadecan-1,3-diol und aus 2-(D,L-Mandeloyl)aminooctadecan-1,3-diol ausgewählt ist.

5. Verfahren zur Herstellung einer Verbindung der Formel (I) gemäß Anspruch 1,
dadurch **gekennzeichnet**, daß man:
(a) in wasserfreiem Medium oder in Gegenwart eines geeigneten Lösungsmittels die Amin-Funktion eines Sphingosin oder Sphinganin der Formel: 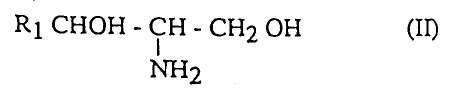 worin R₁ ein C₁₁₋₂₁-Alkyl- oder -Alkenylrest ist, wobei diese Verbindung eine razemische Mischung aus Erythro- und Threo-Diastereoisomeren in Erythro-Threo-Verhältnissen von 85:15 bis 20:80 und vorzugsweise von 65:35 bis 45:55 darstellt, mit einem Acylierungsmittel acyliert, das aus Säurechloriden, Säureanhydriden, gemischen Anhydriden, p-Nitrophenolestern, Estern von Succinimid, Estern von Carbodiimid, C₁₋₈-Niedrigalkylestern, Azoliden und aus O-Carboxyanhydriden der entsprechenden 2-Hydroxisäuren ausgewählt ist, um eine Verbindung der Formel zu erhalten: 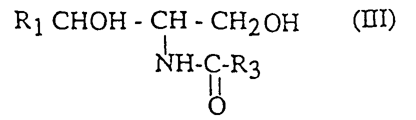 worin R₁ wie vorstehend definiert ist und R₃ entweder den Rest R₂ oder einen Rest darstellt, der aus linearen oder verzweigten C₁₋₂₉- und vorzugsweise aus linearen C₁₋₂₁-Alkyl- oder aus C₇₋₂₉- und vorzugsweise aus C₇₋₁₉-Aralkylresten ausgewählt ist, welche in α-Position zur Carbonylgruppe substituiert sind, wobei der Substituent aus den Resten -Br, -Cl, -J und aus -OB ausgewählt ist, wobei -OB eine zur Bildung einer -OH-Gruppe geeignete Gruppe ist,
(b) die Verbindung der Formel (III) gewinnt, und daß man
(c) gegebenenfalls, wenn sich R₃ von R₂ unterscheidet, die erhaltene Verbindung der Formel (III) hydrolysiert, um die Gruppe -OB in eine Hydroxylgruppe zu überführen.

6. Verfahren gemäß Anspruch 5,
dadurch **gekennzeichnet**, daß man nach Stufe (a) die Acylierung der Amin-Funktion des Sphingosin oder Sphinganin der Formel (II) und vor der Stufe (b) der Gewinnung der Verbindung der Formel (III) eine Stufe durchführt, um Hydroxylgruppen zu schützen, wobei man in der aus Stufe (a) sich ergebenden Mischung ein Schutzmittel für -OH-Gruppen reagieren läßt, das aus Säureanhydriden, Säurehalogeniden und Chlorsilanen ausgewählt ist, und daß man nach Stufe (b) der Gewinnung der Verbindung der Formel (III) die Verbindung der Formel (III) hydrolysiert, vorzugsweise in basischem Milieu, und die Verbindung der Formel (I) gewinnt.

7. Verfahren gemäß Anspruch 6,
dadurch **gekennzeichnet**, daß das Schutzmittel der Hydroxylgruppen aus Acetanhydrid, Acetylchlorid, Benzoylchlorid, Benzylchlorid, Benzylbromid und aus Chlorsilanen der Formeln Cl(Si(CH₃)₃, ClSi(CH₃)₂(tBu) und ClSi(tBu)(-C₆H₅)₂ ausgewählt ist.

8. Verfahren gemäß einem der Ansprüche 5 bis 7,
dadurch
**gekennzeichnet**, daß das Acylierungsmittel die Formel R₃COA (IV) aufweist, worin R₃ darstellt:
- entweder den Rest R₂
- oder einen Rest, ausgewählt aus linearen oder verzweigten C₁₋₂₉- und vorzugsweise linearen C₁₋₂₁-Alkyl- und aus C₇₋₂₉- und vorzugsweise C₇₋₁₉-Aralkylresten, welche in α-Position zur Carbonylgruppe substituiert sind, wobei der Substituent in α-Position zur Carbonylgruppe aus den Resten -Br, -Cl, -J und aus -OB ausgewählt ist, worin -OB eine zur Bildung einer -OH-Gruppe geeignete Gruppe ist,
und worin A ausgewählt ist aus Halogenen, 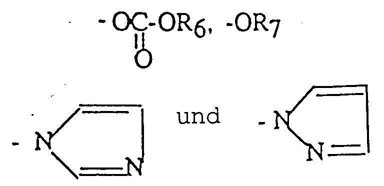 worin R₆ ein C₂₋₈-Niedrigalkylrest ist und R₇ ausgewählt ist aus C₁₋₈-Niedrigalkylresten, 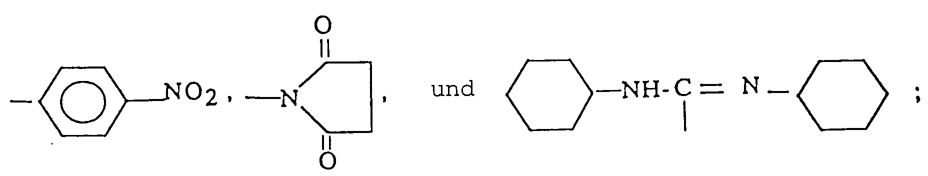 mit der Maßgabe, daß, wenn R₃ den Rest R₂ darstellt, A sich von -Cl unterscheidet.

9. Verfahren gemäß Anspruch 8,
dadurch **gekennzeichnet**, daß der Rest -OB des Acylierungsmittels aus Acetat-, Benzoat-, Benzyloxi-, -OSi(CH₃)₃, -OSi(Ch₃)₂(tBu), -OSi(tBu)(-C₆H₅)₂ ausgewählt ist.

10. Verfahren gemäß Anspruch 8,
dadurch **gekennzeichnet**, daß das Acylierungsmittel aus 2-Hydroxihexadecanoat von Succinimid, 2-Hydroxihexadecanoat von Dicyclohexylcarbodiimid, 2-Hydroxidocosanoat von Succinimid, 2-Hydroxidocosanoat von Dicylcohexylcarbodiimid und aus D,L-Mandeloat von Sicciniumid ausgewählt ist.

11. Verfahren gemäß Anspruch 10,
dadurch **gekennzeichnet**, daß die Verbindung der Formel (II) 2-Aminooctadecan-1,3-diol ist.

12. Zusammensetzung zur kosmetischen oder
dermopharmazeutischen Verwendung,
dadurch **gekennzeichnet**, daß
sie eine Verbindung der Formel (I) gemäß einem der Ansprüche 1 bis 4 enthält.

13. Zusammensetzung gemäß Anspruch 12,
dadurch **gekennzeichnet**, daß sie 0,05 bis 20 und vorzugsweise 0,01 bis 10 Gew.% Verbindung der Formel (I) enthält.

14. Zusammensetzung gemäß Anspruch 12 oder 13,
dadurch **gekennzeichnet**, daß sie ausserdem einen Hilfsstoff aufweist, ausgewählt aus Fettkörpern, Lösungsmitteln, Wasser, Verdickungsmitteln, Emulgatoren, hydratisierenden Produkten, Weichmachern, Sonnenfilterstoffen, Germiziden, Farbstoffen, Konservierungsstoffen, Parfum-Produkten, Treibmitteln und aus oberflächenaktiven Mitteln.

15. Zusammensetzung gemäß einem der Ansprüche 12 bis 14,
dadurch **gekennzeichnet**, daß sie in Form einer Emulsion vorliegt, deren Fettphase, die 5 bis 60 % des Gesamtgewichts der Emulsion darstellt, im wesentlichen aus einer Mischung aus Verbindungen der Formel (I) mit mindestens einem Öl zusammengesetzt ist, wobei die wässrige Phase 30 bis 85% des Gesamtgewichts der Emulsion darstellt, und wobei der Emulgator mit 1 bis 20 und vorzugsweise mit 2 bis 12 Gew.% vorhanden ist, bezogen auf das Gesamtgewicht der Emulsion.

16. Zusammensetzung gemäß enem der Ansprüche 12 bis 14,
dadurch **gekennzeichnet**, daß sie in Form einer öligen, oleoalkoholischen oder hydroalkoholischen Lotion, in Form eines Gels, einer Dispersion oder von Feststoffstäbchen, eines Spray oder eines Aerosol-Schaums vorliegt.

17. Zusammensetzung gemäß einem der Ansprüche 12 bis 14,
dadurch **gekennzeichnet**, daß sie in Form einer wässrigen Dispersion aus lipidischen Kügelchen aus molekularen Schichten vorliegt, die so aufgebaut sind, daß sie eine wässrige eingekapselte Phase umschließen, wobei diese Schichten aus mindestens einer Verbindung der Formel (I) in Abmischung mit mindestens einer weiteren lipidischen Verbindung zusammengesetzt sind.

18. Zusammensetzung in Form einer wässrigen Dispersion aus lipidischen Kügelchen gemäß Anspruch 17,
dadurch **gekennzeichnet**, daß die weitere lipidische Verbindung aus langkettigen Alkoholen und Diolen, Sterolen, Phospholipiden, Glycolipiden, Cholesterylsulfaten und -phosphaten, langkettigen Aminen und deren quaternären Ammoniumderivaten, Dihydroxialkylaminen, polyoxethylierten Fettaminen, langkettigen Aminoalkoholestern, deren Salzen und quaternären Ammoniumderivaten, Phosphorestern von Fettalkoholen, Alkylsulfaten und aus Fettsäure in Form von Salzen ausgewählt ist.

19. Zusammensetzung in Form einer wässrigen Dispersion aus lipidischen Kügelchen gemäß Anspruch 17 oder 18,
dadurch **gekennzeichnet**, daß die Kügelchen einen Durchmesser von 0,05 bis 5 µm aufweisen.

20. Zusammensetzung in Form einer wässrigen Dispersion aus lipidischen Kügelchen gemäß einem der Ansprüche 17 bis 19,
dadurch **gekennzeichnet**, daß die in den Kügelchen eingekapselte wässrige Phase Wasser oder eine wässrige Lösung einer Aktivsubstanz ist.

21. Zusammensetzung in Form einer wässrigen Dispersion aus lipidischen Kügelchen gemäß einem der Ansprüche 17 bis 20,
dadurch **gekennzeichnet**, daß die in den Kügelchen eingekapselte wässrige Phase mindestens eine wasserlösliche kosmetische und/oder pharmazeutische Aktivsusbtanz enthält, ausgewählt aus Feuchtigkeitsmitteln, Mitteln zur künstlichen Bräunung, gegebenenfalls zusammen mit Färbemitteln, wasserlöslischen Sonnenfilterstoffen, Antischwitzmitteln, Deodorantien, Astringentien, Erfrischungsprodukten, Tonika, Vernarbungsmitteln, Keratolytika, Enthaarungsmitteln, parfürmierten Wässern, Extrakten von pflanzlichen Geweben, wasserlöslichen Farbstoffen, Antischuppenmitteln, Seborrheika, Oxidations-, Reduktionsmitteln, Vitaminen, Hormonen, Enzymen, Impfstoffen, entzündungshemmenden Mitteln, Antibiotika, Bakteriziden und aus zytotoxischen oder antitumoralen Mitteln.

22. Zusammensetzung in Form einer wässrigen Dispersion aus lipidischen Kügelchen gemäß einem der Ansprüche 17 bis 20,
dadurch **gekennzeichnet**, daß sie mindestens eine fettlöslische Aktivsubstanz enthält, ausgewählt aus fettlöslichen Sonnenfilterstoffen, Substanzen zur Verbesserung des Zustands trockener oder seniler Hautbereiche, Tocopherolen, Vitaminen E, F oder A und deren Estern, Retinoesäure, Antioxidantien, essentiellen Fettsäuren, Glycylrhetinsäure, Keratolytika und aus Karotinoiden.

23. Zusammensetzung in Form einer wässrigen Dispersion aus lipidischen Kügelchen gemäß einem der Ansprüche 17 bis 22,
dadurch **gekennzeichnet**, daß sie 2 bis 70 Gew.% einer flüssigen, mit Wasser nicht mischbaren Phase enthält, ausgewählt aus Ölen, Kohlenwasserstoffen, halogenierten Wasserstoffen, Perfluortributylamin, Polysiloxanen, Estern organischer Säuren, Ethern und aus Polyethern.

24. Verwendung einer Verbindung der Formel (I) gemäß einem der Ansprüche 1 bis 4 in der Kosmetik.

25. Verwendung der Verbindung der Formel (I) gemäß einem der Ansprüche 1 bis 4 als wachsartiger Bestandteil mit weichmachenden und glättenden Eigenschaften in Emulsionen, Dispersionen, Gelen, Feststoffstäbchen oder in Lotionen zur kosmetischen Verwendung.

26. Verwendung der Verbindung der Formel (I) gemäß einem der Ansprüche 1 bis 4 in Abmischung mit mindestens einer weiteren lipidischen Verbindung zur Bildung von Dispersionen aus lipidischen Kügelchen zur kosmetischen Verwendung.

27. Verfahren zur hydratisierenden kosmetischen Behandlung der Haut, der Haare oder von Behaarungen,
dadurch **gekennzeichnet**, daß man auf die Haut, die Haare oder die Behaarungen eine ausreichende Menge einer Zusammensetzung gemäß einem der Ansprüche 12 bis 23 aufträgt und zur Anwendung bringt.
